# EUROPEAN PATENT APPLICATION

(11) **EP 4 835 466 A1**
(43) Date of publication of application: **04.11.2026**
(21) Application number: 23963107.0
(22) Date of filing: 27.12.2023
(51) Int. Cl.: G01N 33/48, G01N 33/50

(54) **SPECIMEN INSPECTION METHOD AND SPECIMEN INSPECTION SYSTEM**

(71) Applicant: Hitachi High-Tech Corporation, Minato-ku Tokyo 105-6409 (JP)
(72) Inventor: FUJIOKA, Michiru, Tokyo 105-6409 (JP)
(74) Representative: MERH-IP Matias Erny Reichl Hoffmann Patentanwälte PartG mbB
(86) International application number: PCT/JP2023/046907
(87) International publication number: WO 2025/141758

(57) **Abstract**

This specimen test method comprises: a prediction step for predicting the amount of gDNA mixed in collected blood on the basis of the number of days elapsed since blood collection and data indicating the relationship between the number of days elapsed since blood collection and the amount of gDNA contained in blood in accordance with the type of at least one blood collection tube; and a determination step for determining a processing step of the collected blood on the basis of the amount of gDNA to be mixed predicted in the prediction step. Thus, it is possible to provide a specimen test method and a specimen test system that achieve accurate and rapid results as compared to prior art by reducing the time until a test result is obtained and the amount of substances required for test as compared to prior art.

## Description

### Technical Field

The present invention relates to a specimen test method and a specimen test system for collecting a nucleic acid from blood and analyzing the nucleic acid.

### Background Art

NPL 1 discloses a result obtained by examining a storage capacity of a blood cell collection tube for preventing gDNA contamination caused by white blood cell rupture and evaluating performance when combined with a high-sensitivity variant detection technique.

### Citation List

### Non Patent Literature

NPL 1: Yunlong Zhao et al., "Performance comparison of blood collection tubes as liquid biopsy storage system for minimizing cfDNA contamination from genomic DNA", J Clin Lab Anal. 2019 Feb; 33(2):e22670.

### Summary of Invention

### Technical Problem

In recent years, information obtained by a nucleic acid analysis such as a cancer genome test using a next generation sequencing (NGS) system or a digital polymerase chain reaction (PCR) has been utilized in various fields such as medical care, clinical test, pharmaceutical industry, and food industry.

In such a nucleic acid analysis, nucleic acid extraction from various biological samples such as blood, tissue, and cultured cells is an essential pretreatment, and it is known that quality of the extracted nucleic acid greatly affects an analysis process.

A method based on a property that a nucleic acid binds to silica in the presence of a chaotropic agent and a method based on a property that a nucleic acid binds to silica in the presence of an organic solvent are generally used as a nucleic acid extraction method instead of methods using phenol, chloroform, and the like, which are hazardous organic solvents.

When these methods are used, a nucleic acid extraction method using a nucleic acid capturing tip including a solid phase containing silica as a nucleic acid capturing carrier, and a method using magnetic beads (nucleic acid capturing carrier) whose surface is covered with silica have been reported. These methods include a step for binding the nucleic acid to the nucleic acid capturing carrier and an elution step for eluting the nucleic acid from the nucleic acid capturing carrier using an elution liquid.

For example, a non-invasive prenatal genetic testing (NIPT) in obstetrics is a useful method for extracting a nucleic acid from blood and detecting a chromosomal abnormality of a fetus.

However, it has been reported that quality of a blood sample in NIPT may affect test accuracy (see, for example, NPL 1).

Specifically, concentrations of free hemoglobin and potassium ions in blood may increase, and it has been reported that these changes may affect the accuracy and reliability of NIPT, which indicates that quality control of a blood sample is important in performing NIPT in a clinical laboratory.

As a process of quality control after a nucleic acid is extracted from blood, a concentration or an amount of a nucleic acid and a content of an impurity are measured by measuring absorbance using a spectrophotometer or by staining the nucleic acid using a fluorescent dye and measuring a fluorescence intensity. An index of chemical purity of a good DNA is that a value of A260/A280 falls within 1.8 to 2.0 and a value of A260/A230 is larger than 1.0.

When a solvent in which DNA is dissolved contains ethylenediaminetetraacetic acid (EDTA), ethanol, phenol, or the like, the reaction of an NGS library preparation reagent may be inhibited. It is necessary to refer to an instruction manual of a manufacturer and take care to ensure that a solvent does not contain a substance presented to prevent contamination.

In some cases, a degradation state of a nucleic acid is checked by observing a length and a length distribution of the nucleic acid using an electrophoresis method.

DNA derived from a cell that is destroyed by the immune system or DNA derived from a cell that has undergone apoptosis (programmed cell death), which are present in blood called cell free DNA (cfDNA) derived from plasma or serum, may be analyzed. In the case of extracted cfDNA, most of the extracted cfDNA has a length of 140 bp to 200 bp. When high-molecular-weight DNA is observed, it may indicate mixing of genome DNA (gDNA) derived from nucleated cells, which is not suitable for a cfDNA analysis.

Since the high-molecular-weight DNA may be fragmented by a physical impact, it is necessary to pay attention during solution mixing, such as gently tapping with a finger. Repeated freezing and thawing are avoided as much as possible. If a storage period or state is not preferable (vibration and long period) after blood collection, gDNA flows out because blood cells are broken.

Various dedicated blood collection tubes are manufactured and sold in order to make blood cells less likely to be ruptured. However, in a current situation, a blood collection tube can store blood cells for about two weeks, which is a limitation.

In general, 1 [ng] to 50 [ng] of cfDNA can be collected from 1 [mL] of plasma. There is a range depending on the progress of pathological conditions, but when the yield is excessively high, it may indicate mixing of gDNA derived from nucleated cells, which is not suitable for a cfDNA analysis. When a frozen DNA stock solution is used for NGS library preparation, it is desirable to perform quantification immediately before use.

Here, when a test is performed by collecting specimens from a place in a test center, it is difficult to perform the test on the day of blood collection, and a certain storage period or conveyance is involved after blood collection. Therefore, although mixing of gDNA is noticed for the first time by performing QC after nucleic acid extraction, since unnecessary nucleic acid extraction and NGS are performed, there is a problem that reagents and the like used for nucleic acid extraction and test and the cost thereof are wasted, and a time loss in which a pathological condition progresses during re-test is very large.

The invention provides a specimen test method and a specimen test system that can obtain an accurate and rapid result as compared with the related art by reducing a time up to when a test result is obtained and an amount of substances required for test as compared with the related art.

### Solution to Problem

In order to solve the above problems, the invention includes a plurality of means, but only one example will be described here. A specimen test method includes: a prediction step for predicting an amount of gDNA mixed in collected blood based on the number of days elapsed since blood collection and data indicating a relationship between the number of days elapsed since blood collection and an amount of gDNA contained in the blood according to a type of at least one blood collection tube; and a determination step for determining a processing step for the collected blood based on the amount of mixed gDNA predicted in the prediction step.

### Advantageous Effects of Invention

According to the invention, it is possible to obtain an accurate and rapid result as compared with the related art by reducing the time until the test result is obtained and an amount of substances required for the test as compared with the related art. Problems, configurations, and effects other than those described above will be clarified by the following description of embodiment.

### Brief Description of Drawings

[FIG. 1] FIG. 1 is a diagram showing an outline of a specimen test system according to Embodiment 1.
[FIG. 2] FIG. 2 is a flowchart showing processing performed by a management system in the specimen test system according to Embodiment 1.
[FIG. 3] FIG. 3 is a diagram showing a relationship between a concentration of gDNA contained in plasma and the number of days elapsed since blood collection for a specimen in a certain blood collection tube.
[FIG. 4] FIG. 4 is a diagram showing a relationship between a concentration of gDNA contained in plasma and the number of days elapsed since blood collection for a specimen in a blood collection tube different from that in FIG. 3.
[FIG. 5] FIG. 5 is a diagram showing a relationship between a concentration of gDNA contained in plasma and the number of days elapsed since blood collection for a specimen in a blood collection tube different from those in FIGS. 3 and 4.
[FIG. 6] FIG. 6 is a diagram showing a relationship between absorbance of plasma and the number of days elapsed since blood collection for a specimen in the blood collection tube in FIG. 3.
[FIG. 7] FIG. 7 is a diagram showing a relationship between absorbance of plasma and the number of days elapsed since blood collection for a specimen in the blood collection tube in FIG. 4.
[FIG. 8] FIG. 8 is a diagram showing a relationship between absorbance of plasma and the number of days elapsed since blood collection for a specimen in the blood collection tube in FIG. 5.
[FIG. 9] FIG. 9 is a diagram showing an example of a correlation between absorbance and an amount of mixed gDNA for each type of blood collection tube.
[FIG. 10] FIG. 10 is a diagram showing a relationship between saturation of a plasma specimen and an amount of gDNA in a blood collection tube.
[FIG. 11] FIG. 11 is a diagram showing a relationship between brightness of a plasma specimen and an amount of gDNA in a blood collection tube.
[FIG. 12] FIG. 12 is a diagram showing an outline of a specimen test system according to Embodiment 2.
[FIG. 13] FIG. 13 is a diagram showing conditions of samples in which an influence of mixing of gDNA on variant detection was examined.
[FIG. 14] FIG. 14 is a diagram showing an evaluation result of a difference in a variant allele frequency (VAF) with respect to an amount of mixed gDNA.
[FIG. 15] FIG. 15 is a diagram showing an evaluation result of a difference in a variant allele frequency (VAF) with respect to an amount of mixed gDNA.
[FIG. 16] FIG. 16 is a diagram showing an evaluation result of a difference in a variant allele frequency (VAF) with respect to an amount of mixed gDNA.
[FIG. 17] FIG. 17 is a diagram showing an outline of a specimen test system according to Embodiment 3.
[FIG. 18] FIG. 18 is a diagram showing an example of an order screen of consumables in the specimen test system according to Embodiment 3.

### Description of Embodiments

Hereinafter, embodiments of a specimen test method and a specimen test system according to the invention will be described with reference to the drawings. In the drawings used in the present description, the same or corresponding components are denoted by the same or similar reference signs, and repeated descriptions of these components may be omitted.

### <Embodiment 1>

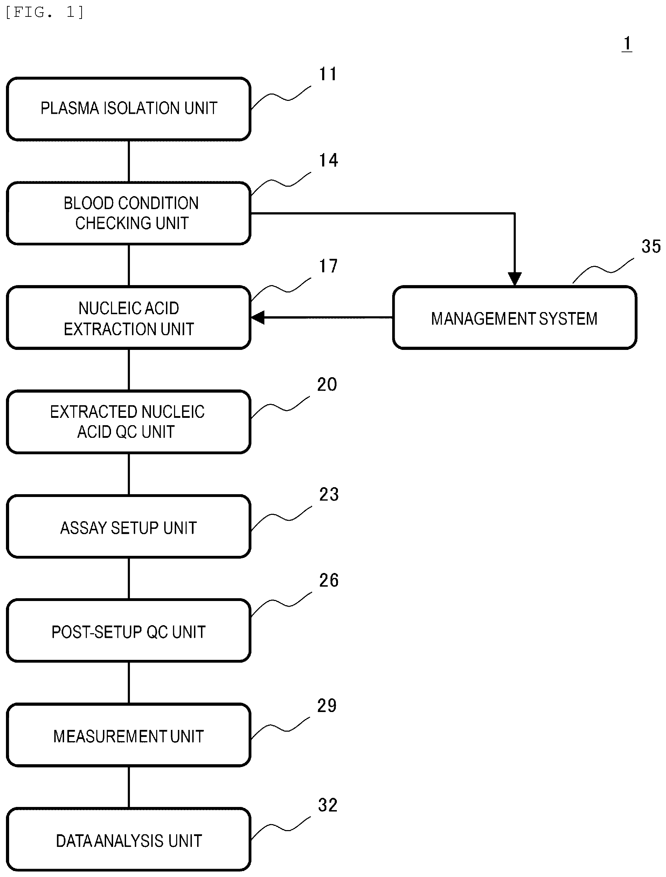

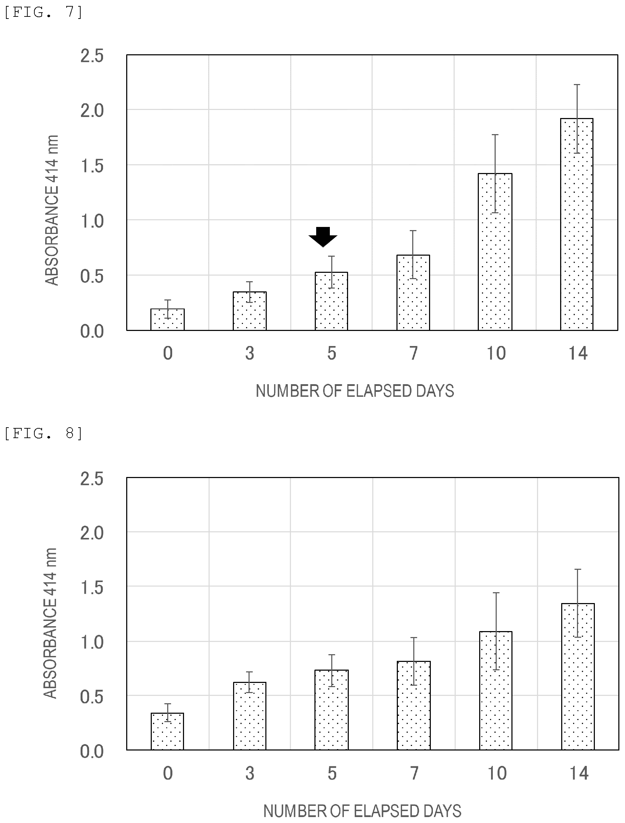

A specimen test method and a specimen test system according to Embodiment 1 of the invention will be described with reference to FIGS. 1 to 11.

First, an overall configuration of the specimen test system that tests a specimen containing blood will be described with reference to FIG. 1. FIG. 1 is a diagram showing an outline of the specimen test system according to Embodiment 1.

A specimen test system 1 shown in FIG. 1 is a system for testing a specimen containing blood, and includes a plasma isolation unit 11, a blood condition checking unit 14, a nucleic acid extraction unit 17, an extracted nucleic acid QC unit 20, an assay setup unit 23, a post-setup QC unit 26, a measurement unit 29, a data analysis unit 32, a management system 35, and the like.

The specimen test system 1 solves a problem that unnecessary NGS or nucleic acid extraction is performed because variant detection sensitivity is lowered due to mixing of gDNA at the time of cfDNA extraction and cfDNA cannot be detected.

More specifically, by measuring color information such as red, green, and blue, or color information such as saturation, hue, and brightness based on an image of a condition of hemolysis in which red blood cells in blood are destroyed and hemoglobin is eluted, and measuring absorbance in the vicinity of 410 nm to 430 nm which is the absorbance of hemoglobin, leakage of gDNA at the time of destruction of white blood cells is indirectly detected based on a condition of elution of the hemoglobin or a condition of destruction of red blood cells, and an amount of unnecessary gDNA contained at the time of nucleic acid extraction or an unnecessary component to be given to an enzyme reaction after nucleic acid extraction is calculated to determine whether a nucleic acid extraction step or a NGS step needs to be performed, thereby solving the above-described problem.

The plasma isolation unit 11 is a mechanism for isolating plasma from blood, and includes, for example, various mechanisms capable of isolating a liquid. The plasma isolation unit 11 is preferably an execution subject of a plasma isolation step for isolating plasma from blood.

The blood condition checking unit 14 is a mechanism for checking a condition of blood in a blood collection tube, and includes, for example, an image sensor such as a CCD camera disposed so as to be able to image the entire blood collection tube, and an illumination for irradiating the blood collection tube with light when imaging the blood collection tube. The illumination is preferably a white or blue light source, but is not limited thereto. The blood condition checking unit 14 is preferably an execution subject of a blood condition checking step for checking a condition of blood.

The nucleic acid extraction unit 17 is a mechanism for extracting a nucleic acid from plasma, and a known configuration can be adopted as the nucleic acid extraction unit 17. The nucleic acid extraction unit 17 is preferably an execution subject of a nucleic acid extraction step for extracting a nucleic acid from plasma.

The extracted nucleic acid QC unit 20 is a mechanism for checking quality of a nucleic acid and obtaining an amount of the nucleic acid in blood based on the quality of the nucleic acid, and includes a configuration for executing fluorescence detection. In addition to the configuration for fluorescence detection, the extracted nucleic acid QC unit 20 includes a configuration for checking a length of the nucleic acid by performing electrophoresis or the like. The extracted nucleic acid QC unit 20 is preferably an execution subject of a nucleic acid quality checking step for checking the quality of the nucleic acid by fluorescence detection, electrophoresis, or real-time PCR and obtaining an amount of the nucleic acid in blood based on the quality of the nucleic acid, or a nucleic acid quality checking step for checking the quality of nucleic acid and calculating an amount of gDNA contained in blood based on the quality of the nucleic acid.

The assay setup unit 23 is a mechanism for setting up a reagent used in a measurement.

The post-setup QC unit 26 is a mechanism for performing checking before a measurement by the measurement unit 29.

The measurement unit 29 is a mechanism that measures a nucleic acid, specifically, at least one of NGS and dPCR, and the measurement unit 29 is preferably an execution subject of a measurement step for measuring a nucleic acid.

The data analysis unit 32 is a mechanism that analyzes a measurement result of at least one of NGS and dPCR. The data analysis unit 32 may be a part of the management system 35.

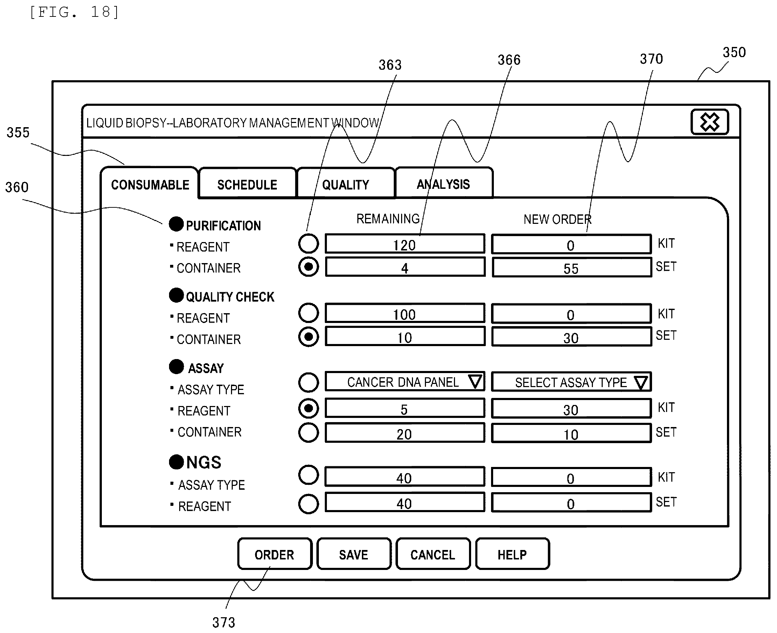

The management system 35 is a part that manages the specimen test system 1 by controlling operations of the plasma isolation unit 11, the blood condition checking unit 14, the nucleic acid extraction unit 17, the extracted nucleic acid QC unit 20, the assay setup unit 23, the post-setup QC unit 26, the measurement unit 29, and the data analysis unit 32, and is implemented by a computer including a display device such as a liquid crystal display (preferably, a request screen 350 in FIG. 18 to be described later is displayed), an input device (preferably, the request screen 350 in FIG. 18 to be described later can be operated), a storage device including a recording medium such as an HDD or an SSD and a controller, a CPU, a memory, and the like. The control of an operation of each device is executed by the management system 35 based on various programs recorded in the storage device.

Operation control processing to be executed by the management system 35 may be integrated in one program, or may be performed by respective programs or a combination thereof. A part or all of the programs may be implemented by dedicated hardware or may be modularized.

In the present embodiment, the management system 35 predicts an amount of gDNA mixed in collected blood based on the number of days elapsed since blood collection and data indicating a relationship between the number of days elapsed since blood collection and an amount of gDNA contained in the blood according to a type of at least one blood collection tube, and determines a processing step in at least one of the plasma isolation unit 11 and the nucleic acid extraction unit 17 based on the predicted amount of mixed gDNA. The "processing step" in the invention includes plasma isolation processing in the plasma isolation unit 11 and nucleic acid extraction processing in the nucleic acid extraction unit 17.

In the present embodiment, the management system 35 predicts the amount of gDNA based on data indicating a relationship between a result obtained by the blood condition checking unit 14 for each type of blood collection tube and an amount of gDNA contained in the blood, and calculates an amount of nucleic acid other than gDNA contained in the blood by subtracting the predicted amount of gDNA from an amount of nucleic acid obtained by a quality checking unit.

Details thereof will be described later.

The management system 35 is an execution subject of a prediction step for predicting an amount of gDNA mixed in collected blood based on the number of days elapsed since blood collection and data indicating a relationship between the number of days elapsed since blood collection and an amount of gDNA contained in the blood according to a type of at least one blood collection tube, and a determination step for determining a processing step for collected blood based on the predicted amount of mixed gDNA in the prediction step, or a prediction step for predicting an amount of gDNA based on data indicating a relationship between a result obtained in the blood condition checking step for each type of blood collection tube and an amount of gDNA contained in the blood, and a nucleic acid amount calculation step for calculating an amount of nucleic acid other than gDNA contained in the blood by subtracting the predicted amount of gDNA in the prediction step from an amount of nucleic acid obtained in the nucleic acid quality checking step.

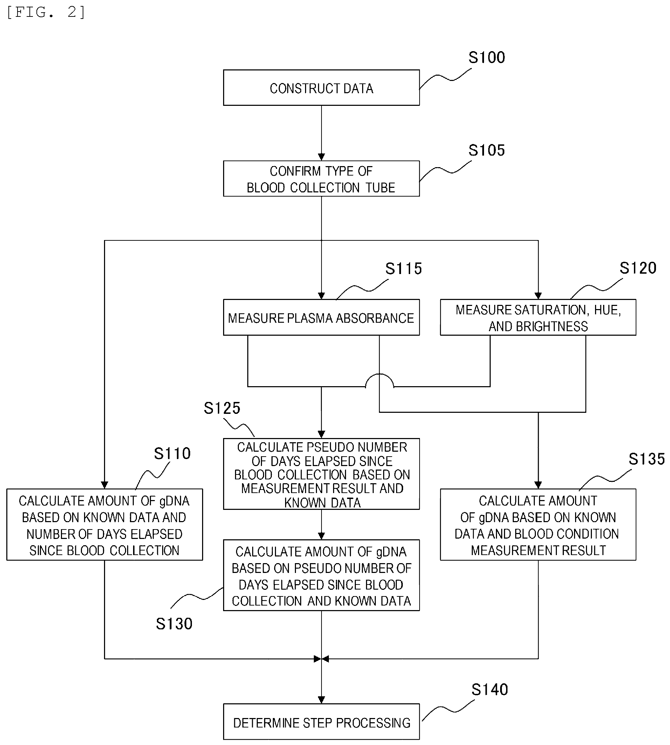

Next, a flow of estimating a mixing state of gDNA based on a condition of whole blood or plasma and determining whether to perform nucleic acid extraction, which is preferably executed by the management system 35 of the specimen test system 1 shown in FIG. 1, will be described with reference to FIG. 2. FIG. 2 is a flowchart showing processing performed by the management system 35 in the specimen test system 1 according to Embodiment 1.

As shown in FIG. 2, first, as a preparation stage, known data is constructed and recorded in the management system 35 (S100).

More specifically, data of the number of days elapsed since blood collection and an amount of mixed gDNA after nucleic acid extraction is acquired as known data for each type of blood collection tube. Since components of an additive for protecting each blood cell component are different depending on a type of blood collection tube, a recommended storage state is different, and thus it is necessary to accumulate data in advance for each type of blood collection tube. The accumulation of the data may be performed by a manufacturer that provides the management system 35 and may be provided to a user, or may be performed independently by the user. Since a storage state, a conveyance condition of a blood collection tube, or performance of a nucleic acid extraction reagent varies depending on each user, management can be performed with higher accuracy by the user.

Next, the management system 35 acquires information on a type of a blood collection tube (S105). Thereafter, the processing proceeds to any one of steps S110, S115, and S120 according to the setting of detection processing.

The acquisition of the information on a type of a blood collection tube in step S105 may be performed in a form in which a user visually inputs a type of a blood collection tube to the system, or may use an image sensor used in a step for checking a blood condition, which is performed in step S115 or step S120 to be described later, and a method capable of determining a type of a blood collection tube is not limited thereto.

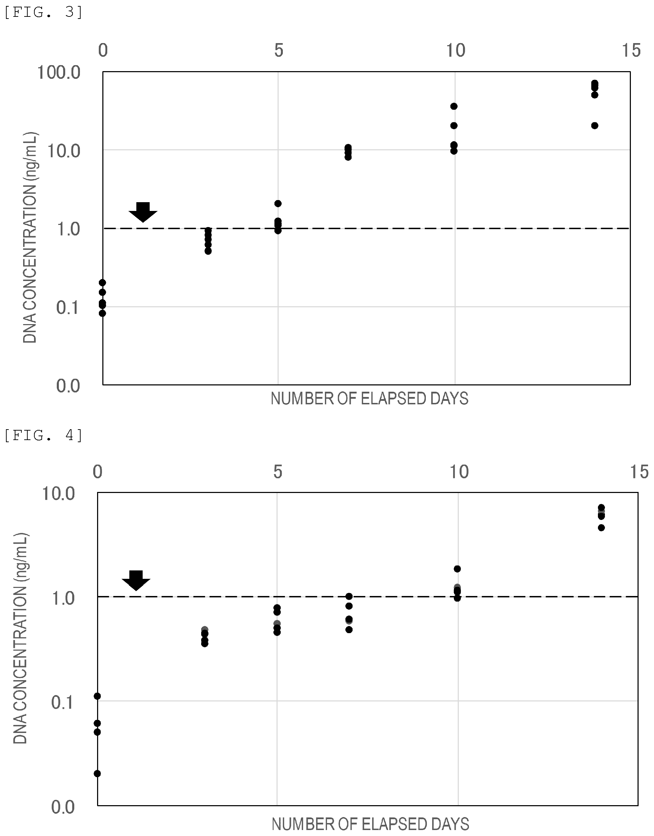

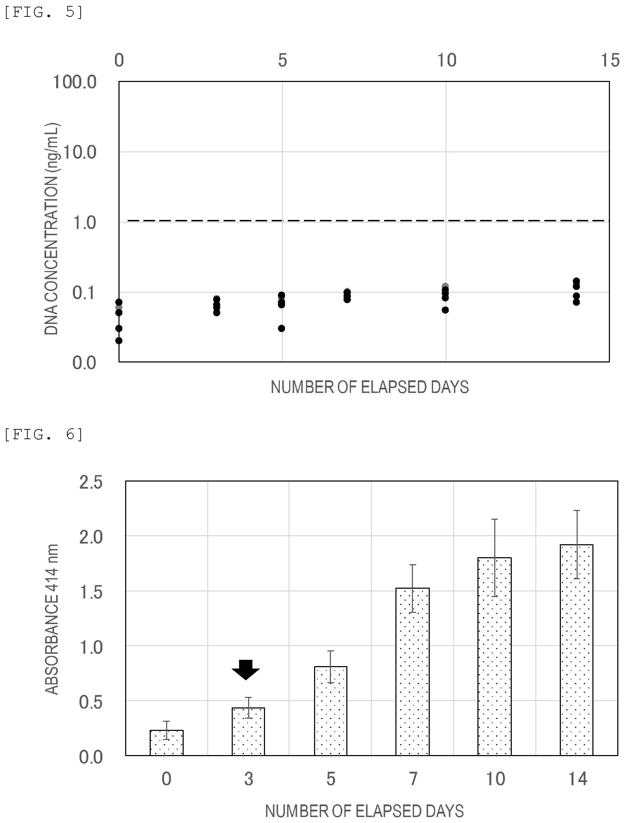

Next, the management system 35 calculates an amount of gDNA based on the known data and the number of days elapsed since blood collection. In the present embodiment, the flow is divided into
1) calculating the amount of gDNA based on data indicating a relationship between the number of days elapsed since blood collection and the amount of gDNA (S110), and
2) checking a blood condition and calculating the amount of gDNA based on data indicating a relationship between the amount of gDNA and the pseudo number of days elapsed since blood collection obtained from a blood condition (S115 or S120 → S125 → S130, or S115 or S120 → S135). This will be described below with reference to FIGS. 3 to 8. FIGS. 3 to 5 show a relationship between a DNA concentration and the number of days elapsed since blood collection for a specimen in a certain blood collection tube, and FIGS. 6 to 8 show a relationship between absorbance and the number of days elapsed since blood collection for specimens in the blood collection tube in FIGS. 3 to 5.

Both 1) and 2) correspond to a prediction step for predicting an amount of gDNA mixed in the collected blood based on data indicating a relationship between the number of days elapsed since blood collection and the amount of gDNA contained in the blood according to a type of at least one blood collection tube, or predicting an amount of gDNA based on data indicating a relationship between a result obtained in the blood condition checking step for each type of blood collection tube and the amount of gDNA contained in the blood.

In this prediction step, the pseudo number of days elapsed since blood collection is obtained based on the data indicating a relationship between a result obtained in the blood condition checking step for each type of blood collection tube and an amount of gDNA contained in the blood, and an amount of gDNA is predicted using the pseudo number of days elapsed since blood collection. Details thereof will be described below.

First, in the case of processing of 1) (S110), the management system 35 obtains the amount of gDNA in blood in a target blood collection tube based on data indicating a relationship between a recorded amount of DNA and the number of days elapsed since blood collection (data as shown in FIGS. 3, 4, and 5), and advances the processing to step S140.

Specifically, the amount of gDNA is calculated using FIG. 3 (data of the number of days elapsed since blood collection and an amount of gDNA) showing data of the number of days elapsed since blood collection associated with a blood collection tube and an amount of gDNA.

For example, it is assumed that an amount of mixed gDNA is to be reduced to 1 [ng] or less per 1 [mL] of plasma, and a dotted line is drawn in FIG. 3 or FIG. 4 at a location serving as a threshold.

Here, since the amount of gDNA to be reduced varies depending on various assays such as NGS and dPCR used in a subsequent step, a step (setting step) of setting a threshold for the amount of gDNA contained in the blood may be performed between a timing aligned with the start of step S100 described above and step S140 to be described later.

An amount of gDNA obtained at a given number of days elapsed since blood collection can be estimated by using an approximate formula such as linear or exponential approximation with respect to the data in FIG. 3 or FIG. 4 indicated by the dotted line.

On the other hand, in the case of processing of 2) (S115 or S120 → S125 → S130, or S115 or S120 → S135), the management system 35 causes the blood condition checking unit 14 to check a condition of blood in a target blood collection tube. A blood condition is checked by performing a measurement (S115) of plasma absorbance or a measurement (S120) of saturation, hue, and brightness. Step S115 or S120 corresponds to the blood condition checking step for checking a condition of blood.

First, the flow (S115) for performing a measurement using absorbance will be described.

The blood condition checking unit 14 measures absorbance of plasma or serum to be analyzed (S115). Prior to the measurement, plasma or serum is obtained by centrifuging collected blood and isolating a supernatant component by the plasma isolation unit 11.

Thereafter, the management system 35 calculates the pseudo number of days elapsed since blood collection in the known data based on a value of absorbance newly measured as an analysis specimen for an approximate formula obtained based on the known number of days elapsed since blood collection and data indicating a relationship between the known number of days elapsed since blood collection such as plasma or serum and the absorbance as shown in any one of FIGS. 6 to 8 (S125). The amount of gDNA is calculated by applying the calculated pseudo number of days elapsed to the approximate formula in FIG. 6 or the like (S130), and the processing proceeds to step S140.

Alternatively, based on a measurement result of the absorbance, the management system 35 calculates the gDNA amount from the known data and a blood condition measurement result based on the data as described in S110 above (S135), and the processing proceeds to step S140.

In this manner, the amount of mixed gDNA can be calculated based on the obtained absorbance.

Next, the flow (S125) performed using saturation, hue, and brightness will be described.

In this case, the blood condition checking unit 14 measures one or more values of saturation, hue, and brightness of plasma or serum to be analyzed (S120). Prior to the measurement, plasma or serum is obtained by centrifuging collected blood and isolating a supernatant component by the plasma isolation unit 11.

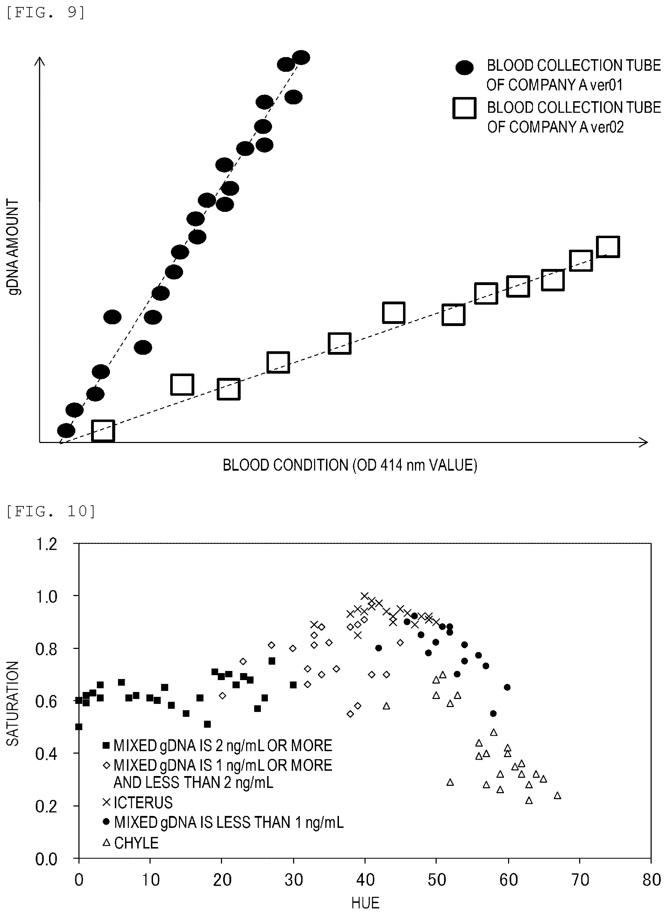

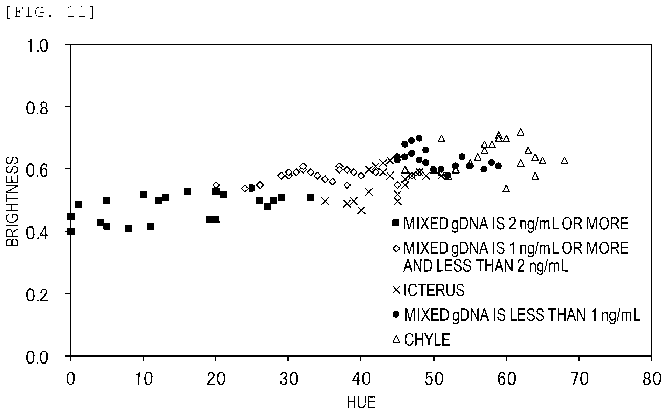

Thereafter, the management system 35 calculates an amount of mixed gDNA based on a relationship between the known "saturation, hue, and brightness" and data of the gDNA amount in FIGS. 10 and 11 (S135), and the processing proceeds to step S140. FIG. 10 is a graph showing a relationship between saturation of a specimen in a blood collection tube and an amount of gDNA, and FIG. 11 is a graph showing a relationship between brightness of a specimen in a blood collection tube and an amount of gDNA.

For example, when it is assumed that an amount of mixed gDNA is to be reduced to 1 [ng] or less per 1 [mL] of plasma, the amount of mixed gDNA is set to 1 [ng/mL] or more for a hue of 45 or less based on the data in FIG. 10. When the processing is performed more strictly, the data in FIG. 11 may also be used, and the hue may be 42 or less and the brightness may be 0.6 or less. The calculation is an example, and a threshold may be set based on a contribution ratio by performing a principal component analysis.

Since the "saturation, hue, and brightness" depend on a measurement environment such as illumination and background color including the relationships shown in FIGS. 10 and 11, it is desirable to unify the measurement environment or acquire the "saturation, hue, and brightness" for each measurement environment.

Alternatively, based on measurement results of the "saturation, hue, and brightness", the management system 35 may calculate the pseudo number of days elapsed since blood collection from the known data and the blood condition measurement result based on the data described in S125 and S130 de(S125), and calculate an amount of gDNA by applying the calculated pseudo number of days elapsed to the approximate formula in FIG. 6 or the like (S130).

Thereafter, the amount of gDNA obtained in any one of the processing of steps S110, S130, and S135 is compared with a specified amount (for example, whether the amount is 1 [ng] or less) to determine a processing step (S140). The step S140 corresponds to a determination step for determining a processing step for the collected blood based on the amount of mixed gDNA predicted in the prediction step, and in this determination step, a processing step can be determined by comparing the amount of mixed gDNA predicted in the prediction step with a threshold.

For example, when it is determined that the amount is less than 1 [ng], plasma is isolated or re-isolated to shift to nucleic acid extraction, and when it is determined that the amount is 1 [ng] or more, abnormality processing may be performed, for example, no nucleic acid is extracted, a nucleic acid is used for other purposes even when a nucleic acid is extracted, or blood is collected again.

Next, in the management system 35, the plasma isolation unit 11 isolates or re-isolates plasma from blood (plasma isolation step), and the nucleic acid extraction unit 17 extracts a nucleic acid from the plasma (nucleic acid extraction step).

A nucleic acid extraction method is not particularly limited, and for example, phenol, chloroform, and the like, which are hazardous organic solvents, were used in the past. In recent years, a method based on a property that a nucleic acid binds to silica in the presence of a chaotropic agent and a method based on a property that a nucleic acid binds to silica in the presence of an organic solvent are generally used.

When these methods are used, a nucleic acid extraction method using a nucleic acid capturing tip including a solid phase containing silica as a nucleic acid capturing carrier, and a method using magnetic beads (nucleic acid capturing carrier) whose surface is covered with silica have been reported. These methods include a step for binding the nucleic acid to the nucleic acid capturing carrier and an elution step for eluting the nucleic acid from the nucleic acid capturing carrier using an elution liquid.

In the method using magnetic beads, after the elution step, the magnetic beads are collected from the elution liquid using a magnet. As an example, there is a method of aspirating an elution liquid containing magnetic beads into a dispensing tip, retaining the magnetic beads in the dispensing tip using a magnet, and discharging only the elution liquid from the dispensing tip. As another example, there is a method of collecting magnetic beads from an elution liquid containing the magnetic beads by inserting a rod-shaped magnet (which may be covered with a cover) into the elution liquid.

In the management system 35, as a process of quality control after a nucleic acid is extracted, the extracted nucleic acid QC unit 20 measures a concentration and an amount of the nucleic acid and a content of an impurity by measuring the absorbance using a spectrophotometer or by staining the nucleic acid using a fluorescent dye and measuring a fluorescence intensity. An amount, a length, purity, and structural integrity can be used as indices for quality control of a nucleic acid. This step corresponds to a nucleic acid quality checking step for checking quality of a nucleic acid and obtaining an amount of a nucleic acid in blood based on the quality of the nucleic acid.

An index of chemical purity of a good DNA is that a value of A260/A280 falls within 1.8 to 2.0 and a value of A260/A230 is larger than 1.0. When a solvent in which a nucleic acid is dissolved contains EDTA, ethanol, phenol, or the like, the reaction of an NGS library preparation reagent may be inhibited. It is necessary to refer to an instruction manual of a manufacturer and take care to ensure that a solvent does not contain a substance presented to prevent contamination.

Since an amount of nucleic acid measured here is the sum of cfDNA and gDNA, an amount of cfDNA may be calculated by subtracting the amount of gDNA predicted in the prediction step. This step corresponds to a nucleic acid amount calculation step for calculating an amount of a nucleic acid other than gDNA contained in blood by subtracting the amount of gDNA predicted in the prediction step from the amount of nucleic acid obtained in the nucleic acid quality checking step.

An amount of mixed gDNA can be checked by, for example, observing a length of a nucleic acid and a length distribution using an electrophoresis method. More specifically, cfDNA has a distribution in which a peak is obtained around 140 bp to 180 bp, and gDNA has a distribution in which a peak is obtained around several tens of kbp. Depending on a degradation state, a length distribution may be less than that. That is, the detection by the electrophoresis method can check a degradation state of a nucleic acid.

There is also a method of evaluating nucleic acid quality by using a Ct value (or a Cq value) obtained by a real-time PCR method as an index of structural integrity. Specifically, primers are prepared and reacted so as to produce two types of PCR amplification products having different lengths. For example, a method is used in which a difference (ΔCt value or ΔCq value) between Ct values obtained from amplicon sizes (for example, short chain amplicon of about 50 bp to 100 bp and long chain amplicon of about 100 bp to 300 bp) is used as an index. It is possible to check structural integrity as to whether a nucleic acid can be used in a later step by checking that an obtained nucleic acid is not amplified when the nucleic acid is degraded for some reason or a double-stranded DNA is partially subjected to a nick (partial fragmentation).

Although several methods for checking quality of a nucleic acid have been described above, the invention is not limited thereto. The quality to be checked is checked using gDNA, but is not limited thereto. An amount, a length, purity, or structural integrity may be determined as an index. When an amount of nucleic acid is insufficient, an expected result cannot be obtained in a subsequent step if a length is short, purity is low, or structural integrity is low. Therefore, an appropriate value of such an index is determined in a step for 0).

Further, quality information of a nucleic acid acquired here can be combined with information of a type of a blood collection tube and a blood condition such as hemolysis shown in FIGS. 3 to 11, and correlated information can be accumulated as accumulated data to update known data.

For example, since a correlation between the absorbance and the amount of mixed gDNA changes in preparation for an update of a blood collection tube not supported by the management system 35 (extension of a storage period due to improvement of an additive or the like), it is possible to support the update of the blood collection tube by newly creating data indicating an example of the correlation between the absorbance and the amount of mixed gDNA for each type of blood collection tube as shown in FIG. 9 and recording the data in a database of the management system 35 also referred to by a customer side.

In this manner, an amount of gDNA contained in a specimen is calculated in the nucleic acid quality checking step, and it is desirable that the nucleic acid quality checking step further includes an improvement step for adding and correcting the data indicating the relationship between the result obtained in the blood condition checking step and the amount of gDNA contained in blood, the data being used in the prediction step. It is desirable that the nucleic acid quality checking step further includes an accumulation step for accumulating data by performing the improvement step for each test community, so that a correlation using data can be calculated and an amount of mixed gDNA can be calculated even in an unknown cfDNA blood collection tube.

Here, the calculated amount of gDNA is compared with the amount of gDNA predicted in the prediction step, and when it is determined that a difference is equal to or larger than a certain threshold, there is a possibility that inappropriate processing is performed in a step after the plasma isolation step.

For example, in a case where collected blood is centrifuged and a supernatant component is isolated, when nucleic acid extraction is performed after a buffy coat containing white blood cells as a main component or a layer of red blood cells, which serves as a lower layer, is aspirated, gDNA is mixed from white blood cells or the like. This is because, for example, a failure of a CCD camera serving as a liquid surface detection mechanism, an illumination or a pressure sensor or a capacitance sensor at the time of image detection, or the like. Even when a liquid surface is correctly detected, there are problems such as malfunction of a mechanism for isolating plasma or serum, non-operation at a correct position, incorrect insertion of a dispensing tip, and an inappropriate aspiration speed.

Therefore, in a case where this step is performed by an automated apparatus, the management system 35 can set a flag to notify a user that there is an abnormality in an apparatus such as an image system that checks dispensing or a height of a liquid phase, and may appropriately instruct the user to check a location of the abnormality. Alternatively, it is also acceptable to directly contact a manufacturer of the automated apparatus. By communicating with the manufacturer, it is possible to perform remote repair and prepare a repair part in advance, and it is possible to quickly perform repair.

This processing corresponds to an output step for comparing the calculated amount of gDNA with the amount of gDNA predicted in the prediction step and outputting occurrence of an abnormality when it is determined that a difference is equal to or larger than a certain threshold, or a step for comparing an amount of gDNA predicted from the number of days elapsed since blood collection with an amount of gDNA predicted using the pseudo number of days elapsed since blood collection and outputting occurrence of an abnormality when it is determined that a difference is equal to or larger than a certain threshold.

Thereafter, the management system 35 causes the assay setup unit 23, the post-setup QC unit 26, the measurement unit 29, and the data analysis unit 32 to perform assay setup and measurement by NGS and dPCR (measurement step for measuring a nucleic acid).

The quality-checked nucleic acid is prepared for an analysis performed by a measurement device such as a next generation DNA sequencer (NGS) or a digital PCR.

For example, when an analysis related to cancer is performed, a portion of a gene sequence related to one or multiple cancers is amplified, and adapters including DNA sequences are provided at both ends. The adapter is used for measurement by the next generation DNA sequencer (NGS) or the digital PCR. Therefore, a type and the number of genes of a gene sequence to be amplified vary depending on a target analysis, and further vary depending on a measurement device for analysis.

At the time of preparation, whether preparation has been appropriately performed is appropriately checked by a step performed by the extracted nucleic acid QC unit 20. For example, it is checked whether there is an appropriate amount of nucleic acid before amplification of a gene sequence, whether there is an appropriate amount of nucleic acid after amplification, whether there is an appropriate amount of nucleic acid when an adapter is provided and the gene sequence is brought into various measurement devices, and the like. When an amount is not an appropriate amount, control may be performed by the management system so that pre-stage processing is re-performed or processing is interrupted as appropriate and an analysis is reliably performed by the measurement device.

Next, effects of the present embodiment will be described.

This specimen test method according to Embodiment 1 of the invention includes: a prediction step for predicting an amount of gDNA mixed in collected blood based on the number of days elapsed since blood collection and data indicating a relationship between the number of days elapsed since blood collection and an amount of gDNA contained in blood according to a type of at least one blood collection tube, and a determination step for determining a processing step for the collected blood based on the amount of mixed gDNA predicted in the prediction step.

The specimen test system 1 that performs a test on a specimen containing blood according to Embodiment 1 of the invention described above includes the plasma isolation unit 11 that isolates plasma from blood, the nucleic acid extraction unit 17 that extracts a nucleic acid from the plasma, the measurement unit 29 that measures the nucleic acid, the data analysis unit 32 that analyzes a measurement result of the measurement unit 29, and the management system 35 that manages the specimen test system, in which the management system 35 predicts an amount of gDNA mixed in the collected blood based on the number of days elapsed since blood collection and data indicating a relationship between the number of days elapsed since blood collection and an amount of gDNA contained in the blood according to a type of at least one blood collection tube, and determines a processing step in one or more of the plasma isolation unit 11 and the nucleic acid extraction unit 17 based on the predicted amount of mixed gDNA.

Accordingly, since an influence on the nucleic acid extraction or a step after the nucleic acid extraction can be estimated based on information such as overview information obtained from a blood specimen, a type of the blood collection tube, and the number of days elapsed since blood collection, the amount of mixed gDNA can be grasped in a stage before performing QC and after the nucleic acid extraction, and thus unnecessary nucleic acid extraction and NGS can be reduced as compared with the related art, and it is possible to avoid waste of costs and test substances and to shorten a time due to re-test.

The method further includes a blood condition checking step for checking a blood condition, and in the prediction step, the pseudo number of days elapsed since blood collection is obtained based on data indicating a relationship between a result obtained in the blood condition checking step for each type of blood collection tube and an amount of gDNA contained in the blood, and an amount of gDNA is predicted using the pseudo number of days elapsed since blood collection, so that the amount of gDNA can be predicted with higher accuracy, and a subsequent step can be more appropriately determined.

The method further includes a setting step for setting a threshold for an amount of gDNA contained in the blood, and in the determination step, a processing step is determined by comparing the amount of mixed gDNA predicted in the prediction step with the threshold, so that the determination can be made according to processing in a subsequent measurement step, and measurement suitable for a user environment can be achieved.

The method further includes a step for comparing the amount of gDNA predicted based on the number of days elapsed since blood collection with the amount of gDNA predicted using the pseudo number of days elapsed since blood collection and outputting occurrence of an abnormality when it is determined that a difference is equal to or larger than a certain threshold, so that it is possible to more quickly take measures for a location where an abnormality occurs, to further prevent a test from being wasted, and to further shorten a time up to when a result is obtained.

### <Embodiment 2>

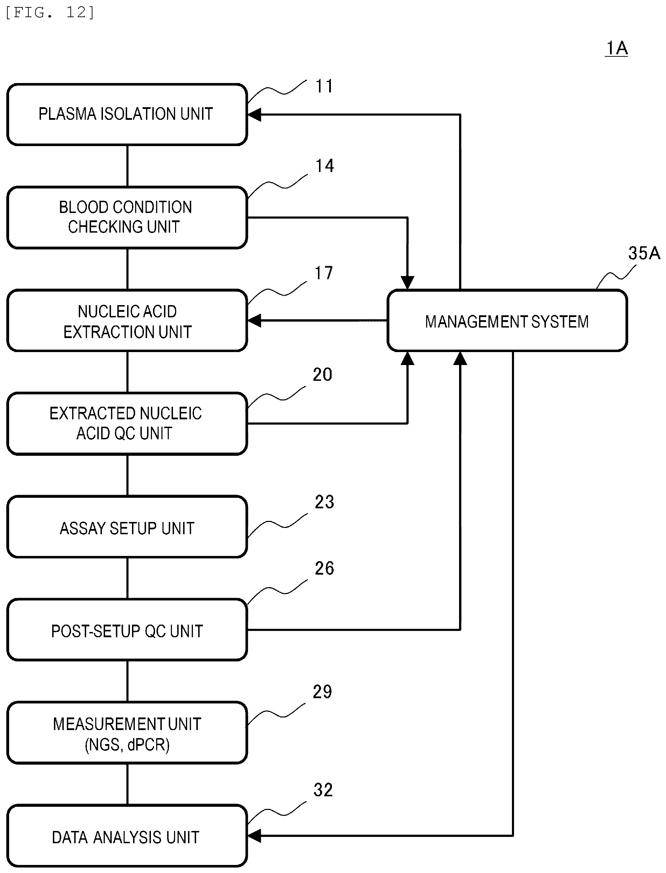

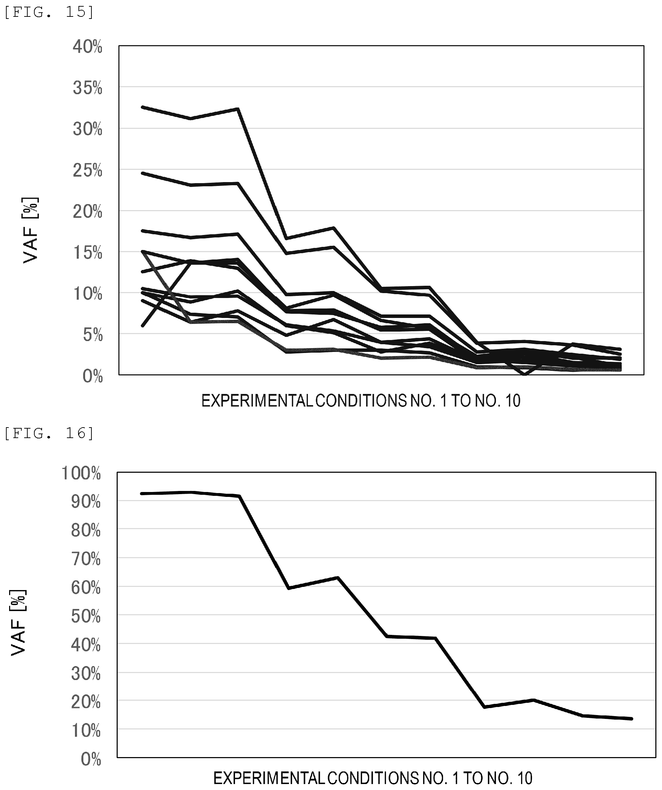

A specimen test method and a specimen test system according to Embodiment 2 of the invention will be described with reference to FIGS. 12 to 16.

A specimen test system 1A according to the present embodiment shown in FIG. 12 includes a management system 35A that calculates a correct variant detection rate by subtracting an influence of gDNA from a variant detection value calculated based on data obtained by NGS or dPCR in a subsequent step, in addition to calculating an amount of mixed gDNA based on information on a blood condition. FIG. 12 is a diagram showing an outline of the specimen test system according to Embodiment 2.

In the management system 35A of the specimen test system 1A according to the present embodiment, an influence of an amount of gDNA predicted using the pseudo number of days elapsed since blood collection can be subtracted from the variant detection value calculated by NGS or dPCR processed in a processing step (correction step). The influence of the amount of gDNA predicted in the prediction step can be subtracted from a measured result (measurement step).

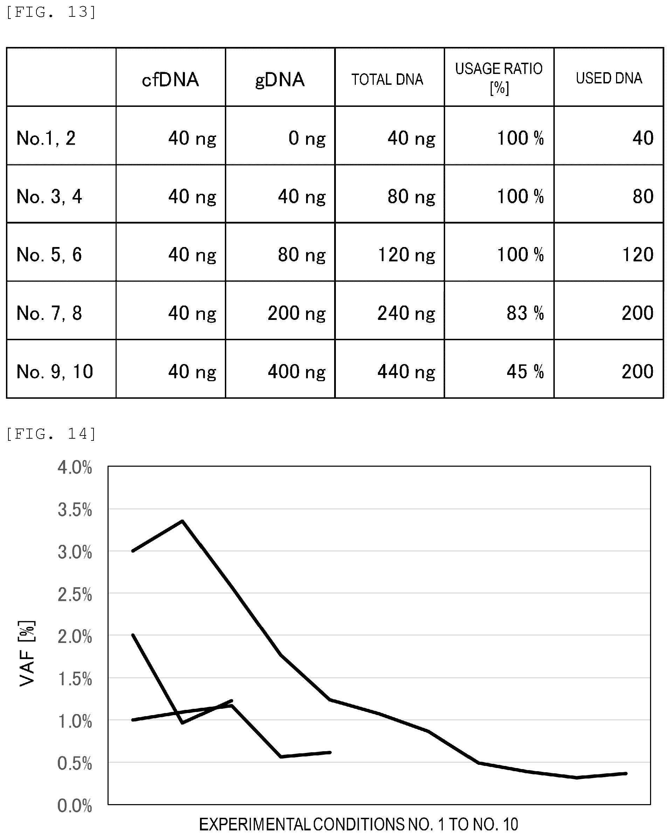

Therefore, first, data about an influence of mixing of gDNA on variant detection is obtained. For example, as shown in FIG. 13, gDNA is mixed in a standard specimen cfDNA by changing an amount or a ratio of gDNA, preparation is performed using an analysis preparation reagent for NGS, and data of a change in variant detection rate when no gDNA is mixed and when gDNA is mixed is acquired using a sequence interpreter.

FIG. 13 is a diagram showing preparation conditions of a sample in which an influence on variant detection due to mixing of gDNA is examined, and FIGS. 14 to 16 are diagrams showing evaluation results of a difference in variant allele frequency with respect to an amount of mixed gDNA.

In FIG. 13, two samples are prepared at one mixing ratio. Under conditions of No. 7 to No. 10, a part of the mixed solution is used with the analysis preparation reagent for NGS.

FIGS. 14 to 16 are divided into three diagrams for each variant allele frequency contained in the standard specimen cfDNA in advance. A horizontal axis represents mixing conditions of gDNA shown in FIG. 13, and a vertical axis represents a variant allele frequency detected by an analysis using a sequence interpreter.

As shown in FIGS. 14 to 16, it can be seen that a variant detection rate decreases as an amount of mixed gDNA increases. In particular, attention should be paid to the fact that, among EGFR genes shown in FIG. 14, a site (base) having a variant allele frequency of 1% or 2% is not detected depending on the amount of mixed gDNA.

For example, it can be seen that early detection of a cancer disease, postoperative examination, and monitoring during treatment are performed by detecting a variant allele frequency of 1%, 2%, or less for a variant serving as a driver gene of the cancer disease, but an appropriate test cannot be performed due to mixing of gDNA. In the data shown in FIG. 14, it can be seen that a variant detection rate at a variant site where a variant allele frequency is 3% is reduced by about half when gDNA of an equivalent amount to cfDNA is mixed.

Such an influence of mixing of gDNA on the variant allele frequency varies depending on an analysis preparation reagent. Therefore, when the same analysis preparation reagent is repeatedly used for analysis, by acquiring such data in advance, the amount of gDNA is estimated based on a condition of blood such as hemolysis, and it is determined whether nucleic acid extraction is to be performed or an NGS analysis is to be performed, so that unnecessary work is not performed. An example of the analysis preparation reagent for NGS has been described above, but the invention is not limited thereto. Similarly, an analysis using a digital PCR or capillary electrophoresis can be applied to all analysis apparatuses affected by a decrease in variant allele frequency due to mixing of gDNA.

Such data is acquired in advance, and an unknown specimen is analyzed.

In the present embodiment, a flow from the acquisition of information on a blood collection tube to a measurement step for measuring a nucleic acid by a measurement device such as a next generation DNA sequencer (NGS) or a digital PCR is the same as that shown in Embodiment 1.

After the measurement step, the management system 35A calculates a variant amount.

The next generation DNA sequencer (NGS) can comprehensively measure a variant based on where the variant is present, the number of molecules with a variant, and the number of molecules without a variant by mapping a read nucleotide sequence to a reference genome sequence.

The digital PCR counts the number of molecules of each gene contained in a prepared sample. Specifically, a sample is distributed to a plurality of wells used for analysis, PCR is performed in these wells, and the presence or absence of a target gene is detected by fluorescence. At this time, some wells contain one or more target genes, and some wells do not contain any target gene. Since a target gene in the sample is randomly distributed, it cannot be checked if only one gene is distributed. Therefore, in addition to calculating a possibility that a plurality of targets are distributed, a correction coefficient is applied using a Poisson model to perform correction instead of simply counting the presence or absence of a target gene, and the number of target genes contained in the sample is quantified, thereby calculating a variant amount and a variant allele frequency of a gene of cancer.

Furthermore, based on the amount of gDNA estimated in a step before the measurement or gDNA actually measured in a quality control step, the management system 35A can calculate how much a variant detection rate decreases with reference to a mixing rate of gDNA acquired in advance as known data and data of an influence on a variant allele frequency.

When a variant detected due to the presence of gDNA of an equivalent amount to cfDNA is reduced by half based on a result of the known data, a variant amount and a variant allele frequency calculated in the assay setup and the measurement step are corrected to a result obtained by reducing by half. The calculation may be performed by calculating an approximate formula of the above data. The approximate formula may be linear or exponential approximation.

Further, the management system 35A can review an isolating condition in the plasma isolation unit 11, can perform maintenance calls for the plasma isolation unit 11, the blood condition checking unit 14, and the like.

Other configurations and operations are substantially the same as those of the specimen test method and the specimen test system according to Embodiment 1 described above, and details thereof are omitted.

In the specimen test method and the specimen test system according to Embodiment 2 of the invention, substantially the same effects as those of the specimen test method and the specimen test system according to Embodiment 1 described above can be obtained.

In addition, the specimen test method further includes a correction step for subtracting an influence of the amount of gDNA predicted using the pseudo number of days elapsed since blood collection from a variant detection value calculated by the NGS or the dPCR, which is to be processed by a processing step including the plasma isolation processing in the plasma isolation unit 11 and the nucleic acid extraction processing in the nucleic acid extraction unit 17, so that it is possible to improve accuracy of determination as to whether a subsequent processing step is performed and what kind of processing step is performed each time a test is repeated, and thus it is possible to construct a test method and a test system capable of achieving daily improvement of test accuracy.

### <Embodiment 3>

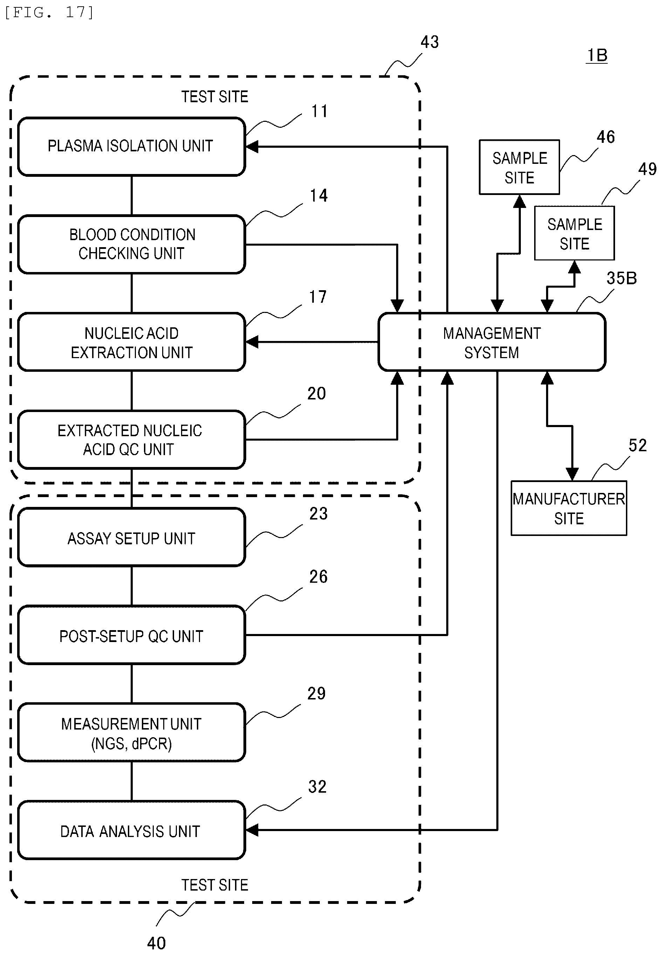

A specimen test method and a specimen test system according to Embodiment 3 of the invention will be described with reference to FIGS. 17 and 18.

In the specimen test system 1 and 1A and the specimen test method according to Embodiment 1, 2, an example is described in which a series of test steps are performed in a system existing in one analysis facility, but in a specimen test system 1B and a specimen test method according to the present embodiment, an aspect is described in which a series of test steps are divided and executed in a plurality of facilities.

FIG. 17 is a diagram showing an outline of the specimen test system according to Embodiment 3. In the specimen test system 1B according to the present embodiment shown in FIG. 17, the plasma isolation unit 11, the blood condition checking unit 14, the nucleic acid extraction unit 17, the extracted nucleic acid QC unit 20, and a part of a management system 35B are provided in a sample site 43 of a hospital or the like, and the assay setup unit 23, the post-setup QC unit 26, the measurement unit 29, the data analysis unit 32, and a part of the management system 35B are provided in a test site 40 of a test operator or the like, and some steps are aggregated in the test site 40. Sample sites 46 and 49 have a configuration equivalent to, larger than, or smaller than that of the sample site 43.

In the embodiment shown in FIG. 17, the management system 35B shares steps up to the extracted nucleic acid QC and a work start and work result in the sample sites 43, 46, and 49 of multiple hospitals or the like via a network, and performs consumable management such as an analysis plan and used consumable prediction in the test site 40.

In the case of a test network in which specimens are collected in the test site 40 as described above, it is complicated to manage information on each specimen (patient information and information on a nucleic acid extraction result). Therefore, the work start and work result in the sample sites 43, 46, and 49 are shared via the network by a function of the management system 35B, which facilitates the analysis plan and the consumable management (used consumable prediction) in the test site 40.

For example, by configuring the management system 35B in a cloud or server format, the management system 35B can be connected, via a network, to the sample sites 43, 46, and 49, the test site 40, and a manufacturer site 52 that manufactures and sells consumables such as reagents and various test components.

Accordingly, a sharing step for sharing a work state and a work result in a processing step via the network, or a sharing step for sharing a work state and a work result in any one or more of the plasma isolation step, the blood condition checking step, the nucleic acid extraction step, the measurement step, the nucleic acid quality checking step, the prediction step, and the nucleic acid amount calculation step via the network, is performed.

In the sharing step, consumable management including an analysis plan and prediction of used consumables can be performed.

Specifically, by sharing an experiment preparation status in the sample sites 43, 46, and 49, when QC indicates qualified, a notification such as an email is sent to the test site 40 of a next step account via the management system 35B of a cloud or server type. Such contact makes it possible to prepare appropriate reagents and consumables in an analysis for dsDNA, ssDNA, RNA, and the like, and to create an efficient analysis plan in advance.

That is, consumables can be checked based on the work start or work result in the sample sites 43, 46, and 49, and in some cases, an alarm can be issued in advance when a reagent or a consumable is insufficient, and an analysis can be performed without delay.

FIG. 18 is a diagram showing an example of an order screen of consumables in the specimen test system according to Embodiment 3. For example, when "consumable" is selected in a tag region 355 of the request screen 350 as shown in FIG. 18, a user can order a consumable on the cloud by selecting a selection region 363 of a target display region 360 for a target reagent or container while checking the number in a remaining amount display region 366, inputting a required number to an order amount display region 370, and pressing "order" in an instruction region 373. The manufacturer site 52 can deliver an ordered consumable to the test site 40 or the sample sites 43, 46, and 49 based on a received order before a required timing.

The management system 35B can also set a flag based on the trend of a result (QC result) in each step. Accordingly, an alert step for setting a flag based on the trend of a relationship between any two of the plasma isolation step, the blood condition checking step, the nucleic acid extraction step, the measurement step, the nucleic acid quality checking step, the prediction step, and the nucleic acid amount calculation step is performed.

For example, it is also possible to set an association flag between patient information (clinical information, other test (tumor marker) result, blood collection tube, blood collection date, blood collection tube conveyance date) and a QC result (nucleic acid extraction result).

More specifically, when there is a correlation among a value of a tumor marker and a blood condition, information on an amount and a length of an extracted nucleic acid, and the like, it is possible to set a flag and notify an analyst. Accordingly, test accuracy can be further improved.

Alternatively, when there is a correlation between a blood collection tube and a poor QC result, it is possible to detect poor compatibility of a nucleic acid extraction reagent with a blood collection tube, or a defect in a lot number of the nucleic acid extraction reagent or a lot number of the blood collection tube. Accordingly, it is possible to interrupt or pay attention to a test of a target blood specimen and quickly perform resampling or the like.

Further, based on information such as a blood collection date, a blood collection tube conveyance date, a blood condition, and an amount and a length of an extracted nucleic acid, it is possible to set a flag when there is a correlation with a poor result such as a poor blood collection condition (insufficient stirring after blood collection) and a poor conveyance condition (temperature, vibration, and the like). Accordingly, it is possible to lead to adoption of a blood collection condition, conveyance, and the like under a more appropriate condition.

Other configurations and operations are substantially the same as those of the specimen test method and the specimen test system according to Embodiment 1 described above, and details thereof are omitted.

In the specimen test method and the specimen test system according to Embodiment 3 of the invention, substantially the same effects as those of the specimen test method and the specimen test system according to Embodiment 1 described above can be obtained.

Further, the specimen test method further includes the sharing step for sharing the work state and the work result in a processing step via the network, so that it is possible to cope with a case where each step is divided for a plurality of facilities.

Further, in the sharing step, by performing the consumable management including an analysis plan and prediction of consumables to be used, the possibility that a test is interrupted due to the shortage of a consumable can be reduced as much as possible, and a test result can be obtained more smoothly.

### <Others>

The invention is not limited to the embodiments described above, and includes various modifications. The embodiments described above have been described in detail to facilitate understanding of the invention, and the invention is not necessarily limited to those including all the configurations described above.

A part of a configuration in one embodiment can be replaced with a configuration in another embodiment, and a configuration in one embodiment can also be added to a configuration in another embodiment. Another configuration may be added to a part of a configuration in each embodiment, and a part of a configuration in each embodiment may also be deleted or replaced with another configuration.

### Reference Signs List

1, 1A, 1B: specimen test system
11: plasma isolation unit
14: blood condition checking unit
17: nucleic acid extraction unit
20: extracted nucleic acid QC unit (quality checking unit)
23: assay setup unit
26: post-setup QC unit
29: measurement unit
32: data analysis unit
35, 35A, 35B: management system
40: test site
43, 46, 49: sample site
52: manufacturer site
350: request screen
355: tag region
360: target display region
363: selection region
366: remaining amount display region
370: order amount display region
373: instruction region

## Claims

1. A specimen test method comprising:
a prediction step for predicting an amount of gDNA mixed in collected blood based on the number of days elapsed since blood collection and data indicating a relationship between the number of days elapsed since blood collection and an amount of gDNA contained in the blood according to a type of at least one blood collection tube; and
a determination step for determining a processing step for the collected blood based on the amount of mixed gDNA predicted in the prediction step.

2. The specimen test method according to claim 1, further comprising:
a blood condition checking step for checking a condition of the blood, wherein
in the prediction step, a pseudo number of days elapsed since blood collection is obtained based on data indicating a relationship between a result obtained in the blood condition checking step for each type of blood collection tube and the amount of gDNA contained in the blood, and the amount of gDNA is predicted using the pseudo number of days elapsed since blood collection.

3. The specimen test method according to claim 2, further comprising:
a setting step for setting a threshold for the amount of gDNA contained in the blood, wherein
in the determination step, the processing step is determined by comparing the amount of mixed gDNA predicted in the prediction step with the threshold.

4. The specimen test method according to claim 2, further comprising:
a step for comparing the amount of gDNA predicted based on the number of days elapsed since blood collection with the amount of gDNA predicted using the pseudo number of days elapsed since blood collection, and outputting occurrence of an abnormality when it is determined that a difference is equal to or larger than a certain threshold.

5. The specimen test method according to claim 2, further comprising:
a correction step for subtracting an influence of the amount of gDNA predicted using the pseudo number of days elapsed since blood collection from a variant detection value calculated by NGS or dPCR, which is to be processed in the processing step.

6. The specimen test method according to claim 1, further comprising:
a sharing step for sharing a work state and a work result in the processing step via a network.

7. The specimen test method according to claim 6, wherein
in the sharing step, consumable management including an analysis plan and prediction of a consumable to be used is performed.

8. A specimen test system for performing a test on a specimen containing blood, the specimen test system comprising:
a plasma isolation unit configured to isolate plasma from the blood;
a nucleic acid extraction unit configured to extract a nucleic acid from the plasma;
a measurement unit configured to measure the nucleic acid;
a data analysis unit configured to analyze a measurement result of the measurement unit; and
a management system configured to manage the specimen test system, wherein
the management system predicts an amount of gDNA mixed in collected blood based on the number of days elapsed since blood collection and data indicating a relationship between the number of days elapsed since blood collection and an amount of gDNA contained in the blood according to a type of at least one blood collection tube, and determines a processing step in one or more of the plasma isolation unit and the nucleic acid extraction unit based on the predicted amount of mixed gDNA.
